# FASCICULE DE BREVET EUROPEEN

(11) **EP 1 049 706 B1**
(45) Date de publication et mention de la délivrance du brevet: **28.05.2003**
(21) Numéro de dépôt: 99900525.9
(22) Date de dépôt: 13.01.1999
(51) Int. Cl.: C07H 15/04, C08B 37/00, A61K 31/70

(54) **NOUVEAUX PENTASACCHARIDES, PROCEDES POUR LEURS PREPARATIONS ET COMPOSITIONS PHARMACEUTIQUES LES CONTENANT**
PENTASACCHARIDE, VERFAHREN ZU IHRER HERSTELLUNG UND PHARMAZEUTISCHE ZUBEREITUNGEN DIE SIE ENTHALTEN
NEW PENTASACCHARIDES, THEIR METHODS OF PRODUCTION AND PHARMACEUTICAL COMPOSITIONS CONTAINING SAME

(30) Priorité: 19.01.1998 FR 9800514
(43) Date de publication de la demande: 08.11.2000
(73) Titulaire: SANOFI-SYNTHELABO, 75013 Paris (FR); Akzo Nobel N.V., 6824 BM Arnhem (NL)
(72) Inventeur: PETITOU, Maurice, F-75645 Paris Cedex 13 (FR)
(74) Mandataire: Thouret-Lemaitre, Elisabeth
(86) Numéro de dépôt international: FR9900045
(87) Numéro de publication internationale: WO99036428

(56) Documents cités:
- WO-A-98/03554
- N.B.SAKURAI ET AL.: "Synthesis of a Conformationally Constrained Heparin-Like Pentasaccharide." CHEMISTRY - A EUROPEAN JOURNAL., vol. 2, no. 8, 1996, pages 1007-1013, XP002080448 ISHERS US
- M.RAGAZZI ET AL.: "Conformation of the Pentasaccharide Corresponding to the Binding Site of Heparin for Antithrombin III." CARBOHYDRATE RESEARCH., vol. 195, no. 2, 1990, pages 169-186, XP002080449 AMSTERDAM NL

## Description

La présente invention concerne des pentasaccharides, leurs procédés de préparation et les compositions pharmaceutiques qui les contiennent.

L'héparine est un polysaccharide de la famille des glycosaminoglycanes, connu pour ses propriétés anti-coagulantes. Il est connu (I. Björk et U. Lindahl, Molecular and Cellular Biochemistry, 1982, Dr, W.Junk Publishers - Pays-Bas) que la coagulation sanguine est un phénomène physiologique complexe. Certains stimuli tels que l'activation de contact et le facteur tissulaire, déclenchent l'activation successive d'une série de facteurs de la coagulation présents dans le plasma sanguin. Quelle que soit la nature du stimulus, les étapes finales sont identiques le facteur X activité (Xa) active le facteur II (également appelé prothrombine), lequel sous sa forme activée (facteur Ila, également appelé thrombine) provoque la protéolyse partielle du fibrinogène soluble avec libération de la fibrine insoluble, un des constituants principaux du caillot sanguin.

Dans les conditions physiologiques normales, l'activité des facteurs de coagulation est régulée par des protéines telles que l'antithrombine III (ATIII) et le cofacteur II de l'héparine (HC II), qui sont également présentes dans le plasma. L'AT III exerce une activité inhibitrice sur un certain nombre de facteurs de coagulation et notamment sur les facteurs Xa et Ila.

L'inhibition du facteur Xa ou du facteur Ila constitue donc le moyen privilégié pour obtenir une activité anticoagulante et antithrombotique puisque ces deux facteurs interviennent dans les deux dernières étapes de la coagulation, lesquelles sont indépendantes du stimulus déclencheur.

Un pentasaccharide tel que celui décrit par P. Sinaÿ *et al*., Carbohydrate Research 1984, *132* C5, représente la séquence minimale de l'héparine, requise pour la liaison à l'AT III. Ce composé a été obtenu il y a environ une quinzaine d'années par synthèse chimique totale.

Depuis, un certain nombre d'oligosaccharides de synthèse, obtenus par synthèse chimique totale et ayant des activités antithrombotiques et anticoagulantes ont été décrits dans la littérature.

Le brevet EP 0 084 999 décrit des dérivés constitués d'unités monosaccharidiques acides uroniques (glucuronique ou iduronique) et glucosamine et possédant des propriétés antithrombotiques intéressantes. En dehors des substituants constitués par des groupes hydroxy, ces composés contiennent des groupes N-sulfate, des groupes N-acétyle et dans certains cas, les groupes anomériques hydroxy sont remplacés par des groupes méthoxy.

La demande EP 0 165134 décrit aussi des oligosaccharides de synthèse ayant des activités antithrombotiques. Ces composés constitués d'unités monosaccharidiques acides uroniques et glucosamine et comportant en position 3 de l'unité glucosamine un groupe O-sulfate, sont aussi décrits dans la demande EP 0 301 618. Ces composés possèdent des propriétés antithrombotiques et anticoagulantes puissantes. Le brevet EP 0 454 220 décrit des dérivés d'acides uroniques et de glucose possédant comme substituants des groupes O-alkyle ou O-sulfate. Ces derniers composés sont aussi dotés de propriétés antithrombotiques et anticoagulantes. Des dérivés glycosaminoglycanoïdes sulfatés dans lesquels les groupes fonctionnels N-sulfate, N-acétate ou hydroxy ont été remplacés par des groupes alcoxy, aryloxy, aralkyloxy ou O-sulfates sont aussi décrits dans le brevet EP 0 529 175. Ces composés ont des propriétés antithrombotiques intéressantes. Ces derniers sont aussi des inhibiteurs de la prolifération des cellules musculaires lisses.

Des oligosaccharides et notamment des pentasaccharides, analogues de la séquence minimale de l'héparine requise pour la liaison à l'AT-III, sont décrits dans Angew. Chem. Int. Ed. Engl. 1993, 32, 3, 434-436. Ces composés contiennent des unités acides glucuroniques ou glucose dont les fonctions hydroxy ont été remplacées par des groupes O-sulfate ou O-méthyle. De nombreuses études ont depuis été réalisées sur des pentasaccharides et il a été indiqué dans la littérature que la conformation de l'unité acide L-iduronique G joue un rôle important pour l'activité des produits. Plusieurs états conformationnels pour l'unité G ont été décrits (⁴*C*₁, ¹*C*₄, ²S₀) et il a été suggéré que cette flexibilité conformationnelle est essentielle pour l'activité biologique de produits contenant de l'acide L-iduronique (B. Casu, M. Petitou, A. Provasoli et P. Sinaÿ, Conformational flexibility : a new concept for explaining binding and biological properties of iduronic acid-containing glycosaminoglycans. Trends Biochem. Sci. 1988, *13*, 221-225). Le document Chem. Eur. J., 1996, 2,8, 1007-1013 divulgue des pentasaccharides dans lesquels l'unité L-iduronique G est dans une conformation contrainte ¹C₄ dite « verrouillée » par un pont alkylène entre l'hydroxyle en position 3 et le carbone en position 5 résultant en une unité 3-O-5-C-alkylene-2-O(R)-α-L-idopyranururonate. Selon ce document, les composés où l'unité L-iduronique G est dans une conformation contrainte ¹C₄ possèdent une très faible activité anti-facteur Xa et donc pas d'affinité pour l'AT III. Ceci montre que la flexibilité conformationnelle de l'unité G est essentielle pour l'activité biologique.

On a maintenant trouvé de manière surprenante qu'en remplaçant un des groupes O-alkyle par un pont alkylène, donc en verrouillant la conformation de l'acide L-iduronique, on obtient des oligosaccharides doués de propriétés biologiques intéressantes bien que dépourvus de flexibilité conformationnelle. En effet, les composés de la présente invention se distinguent des autres héparinoïdes de synthèse décrits dans la littérature, par leur structures originales et par leurs propriétés biologiques puissantes et inattendues. Les composés de l'invention sont des pentasaccharides dont l'unité L-iduronique G est dans la conformation ²S₀ dite "verrouillée" et dont l'unité acide D-glucuronique E possède éventuellement en position 5 un groupe éthyle. Ces composés possèdent une très grande activité anti-facteur Xa, et une grande affinité pour l'AT III.

La présente invention a plus particulièrement pour objet, un pentasaccharide sous forme acide et ses sels pharmaceutiquement acceptables, avec un ou plusieurs cations pharmaceutiquement acceptables, dont la forme anionique a pour formule (I) : 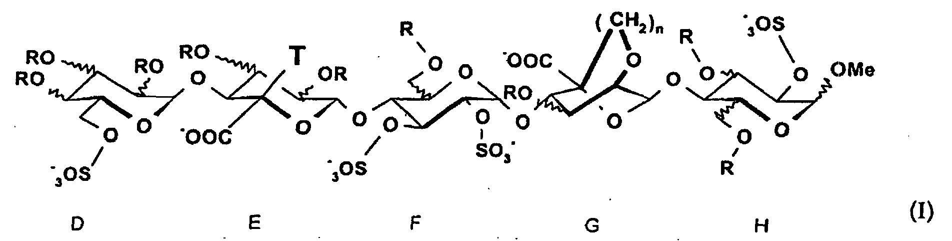 dans laquelle :
- R représente l'hydrogène, un groupe -SO₃⁻, (C₁-C₃) alkyle ou (C₂-C₃) acyle ;
- T représente l'hydrogène ou un groupe éthyle ;
- n représente 1 ou 2.

L'invention englobe les pentasaccharides sous forme acide, ou sous forme de sel pharmaceutiquement acceptable. Dans la forme acide, les fonctions -COO⁻ et -SO₃⁻ sont respectivement sous forme -COOH et -SO₃H.

On entend par sel pharmaceutiquement acceptable des pentasaccharides de l'invention, les pentasaccharides dans lesquels une ou plusieurs des fonctions -COO⁻ ou/et -SO₃⁻ sont liées de façon ionique à un cation métallique pharmaceutiquement acceptable.

Les sels préférés selon l'invention sont ceux dont le cation est choisi parmi les cations des métaux alcalins et plus préférablement encore, ceux dont le cation est Na⁺ ou K⁺.

La présente invention a également pour objet un procédé pour la préparation des pentasaccharides de l'invention, caractérisé en ce que l'on prépare un précurseur de l'unité G que l'on couple sur un précurseur de l'unité H pour obtenir un précurseur de GH, le pentasaccharide étant finalement obtenu : soit en couplant un précurseur de GH sur un précurseur de DEF, soit en couplant un précurseur de GH sur un précurseur de EF puis addition de D.

On peut utiliser n'importe quel précurseur de G, de H, de EF ou de DEF. Ceci signifie que l'on peut, selon ces procédés préparer toute une famille de pentasaccharides ayant en commun l'unité G de configuration verrouillée.

Le procédé décrit ci-dessus est le procédé préféré de l'invention. Toutefois, les pentasaccharides de l'invention peuvent être préparés par d'autres méthodes connues de la chimie des sucres, et notamment, en faisant réagir un monosaccharide comportant des groupes protecteurs tels que décrits par T.W. Green, dans Protective Groups in Organic Synthesis (Wiley, N.Y. 1981) sur les radicaux hydroxy et éventuellement sur les radicaux carboxy s'il en dispose, avec un autre monosaccharide protégé, pour former un disaccharide sur lequel ensuite, on fait réagir un autre monosaccharide protégé, pour former un trisaccharide protégé à partir duquel, on peut obtenir un tetrasaccharide protégé puis un pentasaccharide protégé.

Les pentasaccharides protégés sont ensuite déprotégés et éventuellement sulfatés ou d'abord partiellement déprotégés puis sulfatés et ensuite déprotégés, pour obtenir les composés de l'invention.

De tels procédés sont connus dans la chimie des glucides, et particulièrement décrits par G. Jaurand *et al*., dans Bioorganic and Medicinal Chemistry Letters 1992, 2, 9, 897-900, par J. Basten *et al*., dans Bioorganic and Medicinal Chemistry Letters 1992, 2, 9, 905-910 et par M. Petitou et C.A.A. van Boeckel dans "Chemical syntesis of heparin fragment and analogues" 203-210 - Progress in the chemistry of organic natural products, Ed. Springer Verlag Wien - N.Y. 1992.

Le procédé décrit ci-dessus permet d'obtenir les composés de l'invention sous forme de sels. Pour obtenir les acides correspondants, les composés de l'invention sous forme de sels, sont mis en contact avec une résine échangeuse de cations sous forme acide.

Les composés de l'invention sous forme d'acides peuvent être ensuite neutralisés par une base pour obtenir un sel souhaité.

Pour ce faire, on peut utiliser toute base minérale ou organique, conduisant à des sels pharmaceutiquement acceptables.

On utilise de manière préférentielle l'hydroxyde de sodium, de potassium, de calcium ou de magnésium. Les sels de sodium et de calcium des pentasaccharides de l'invention sont les sels préférés.

Les composés objet de la présente invention, ont des propriétés pharmacologiques et biochimiques intéressantes. Ils possèdent plus particulièrement une grande activité anti-facteur Xa, et une grande affinité pour l'AT III.

Comme cela a été mentionné précédemment , le facteur Xa active dans la cascade de la coagulation la prothrombine en thrombine, qui protéolyse le fibrinogène soluble avec libération de la fibrine insoluble, constituant principal du caillot sanguin.

L'inhibition du facteur Xa constitue donc un moyen privilégié pour obtenir une activité anticoagulante et antithrombotique. L'activité anti-facteur Xa des produits de l'invention a été évaluée à pH 7, selon la méthode décrite par Teien A.N. et Lie M., dans Thrombosis Research 1977, *10,* 399-410, et il a été démontré que les produits de l'invention possèdent une activité anti-Xa égale ou supérieure à celle des héparinoïdes de synthèse déjà connus.

L'affinité des pentasaccharides de l'invention, dont l'anion a pour formule (I), pour l'AT III, a été déterminée par spectrofluorométrie, dans les conditions décrites par D. Atha *et al*. dans Biochemistry 1987, 26, 6454-6461. Les résultats des essais ont démontré que les composés de l'invention possèdent une très grande affinité pour l'AT III.

Par ailleurs, l'activité antithrombotique globale de ces composés a été évaluée chez le rat par un modèle de stase veineuse et induction par la thromboplastine, selon la méthode décrite par J. Reyers *et al*. dans Thrombosis Research 1980, *18,* 669-674.

La DE₅₀ des composés de l'invention est au moins du même ordre ou inférieure à celle des autres héparinoïdes de synthèse déjà connus. Les composés de l'invention présentent donc une spécificité d'action et une activité anticoagulante et antithrombotique intéressantes.

Les composés de l'invention sont utiles pour la préparation de compostions pharmaceutiques administrables par voie parentérale.

Les composés de l'invention sont très peu toxiques ; leur toxicité est parfaitement compatible avec leur utilisation comme médicaments.

Les composés de l'invention sont très stables et sont donc ainsi particulièrement appropriés pour constituer le principe actif des médicaments.

L'invention s'étend aussi aux compositions pharmaceutiques contenant comme principe actif, un composé selon l'invention ou l'un de ses sels pharmaceutiquement acceptables, éventuellement en association avec un ou plusieurs excipients inertes et appropriés.

Dans chaque unité de dosage, le principe actif est présent dans les quantités adaptées aux doses journalières envisagées. Chaque unité de dosage contient de 0,1 à 100 mg de principe actif, de préférence de 0,5 à 50 mg.

Les composés selon l'invention peuvent également être utilisés en association avec un ou plusieurs autres principes actifs utiles pour la thérapeutique souhaitée tels que par exemple des antithrombotiques, des anticoagulants, des antiagrégants plaquettaires tels que par exemple le dipyridamole, l'aspirine, la ticlopidine, le clopidogrel ou des antagonistes du complexe de la glycoproteine IIb/IIIa.

Les compositions pharmaceutiques sont formulées pour l'administration aux mammifères, y compris l'homme, pour le traitement des maladies susdites.

Les compositions pharmaceutiques ainsi obtenues sont présentées avantageusement sous des formes diverses, telles que par exemple, des solutions injectables ou buvables, dragées, comprimés ou gélules. Les solutions injectables sont les formes pharmaceutiques préférées. Les compositions pharmaceutiques de la présente invention sont notamment utiles pour le traitement à titre préventif ou curatif, des troubles de la paroi vasculaire, tels que l'athérosclérose, les états d'hypercoagulabilité observés par exemple à la suite d'opérations chirurgicales de développement tumoraux ou de dérèglements de la coagulation, induits par des activateurs bactériens, viraux, ou enzymatiques.

Plus généralement, les pentasaccharides de l'invention sont utilisables dans le traitement des pathologies dépendantes d'un dysfonctionnement de la coagulation.

La posologie peut largement varier en fonction de l'âge, du poids et de l'état de santé du patient, de la nature et de la sévérité de l'affection, ainsi que de la voie d'administration. Cette posologie comprend l'administration d'une ou plusieurs doses d'environ 0,5 mg à 1000 mg par jour, de préférence d'environ 1 à 100 mg par jour, mieux encore d'environ 0,5 à 50 mg par jour, par exemple de l'ordre de 20 mg par jour, par voie intramusculaire ou par voie sous cutanée, en administrations discontinues ou à intervalles réguliers, ou d'une dose journalière de l'ordre de 200 mg à 1000 mg par jour par voie orale.

Ces doses peuvent naturellement être ajustées pour chaque patient, en fonction des résultats observés et des analyses de sang préalablement effectuées.

L'administration par voie sous-cutanée est la voie préférée.

La présente invention a donc également pour objet les compositions pharmaceutiques qui contiennent à titre de principe actif un des composés ci-dessus éventuellement en association avec un autre principe actif. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, transmuqueux, locale ou rectale, l'ingrédient actif peut être administré sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Pour une administration transmuqueuse le principe actif peut être formulé en présence d'un promoteur tel qu'un sel biliaire, d'un polymère hydrophile tel que par exemple l'hydroxypropylcellulose, l'hydroxypropylméthylcellulose, l'hydroxyéthylcellulose, l'éthylcellulose, la carboxyméthylcellulose, le dextran, la polyvinylpyrrolidone, les pectines, les amidons, la gélatine, la caséine, les acides acryliques, les esters acryliques et leurs copolymères, les polymères ou copolymères de vinyle, les alcools vinyliques, les alcoxypolymères, les polymères d'oxyde de polyéthylène, les polyéthers ou leur mélange.

Le principe actif peut être formulé également sous forme de microcapsules, éventuellement avec un ou plusieurs supports ou additifs.

Le principe actif peut être également présenté sous forme de complexe avec une cyclodextrine, par exemple α, β ou y cyclodextrine, 2-hydroxypropyl-β-cyclodextrine ou méthyl-β-cyclodextrine.

Le principe actif peut également être libéré par un ballonnet le contenant ou par un extenseur endovasculaire introduit dans les vaisseaux sanguins. L'efficacité pharmacologique du principe actif n'est ainsi pas affectée.

L'administration par voie sous-cutanée est la voie préférée.

Les méthodes, les préparations et les schémas suivants illustrent la synthèse des différents intermédiaires utiles à l'obtention des pentasaccharides selon l'invention.

Les abréviations suivantes sont utilisées :
TBDMS : *tert*-butyldiméthylsilyle ; Lev : lévulinyle ; Bn : benzyle ; Bz : benzoyle ; CCM : chromatographie sur couche mince ; Olm : trichloroacétimidyle ; LSIMS : est le sigle anglais de *Liquid Secondary Ion Mass Spectrometry* ; ESIMS : est le sigle anglais de *Electron Spray Ionisation Mass Spectrometry* ; TMS : triméthylsilyle ; TSP : triméthylsilyle tétradeuterio propionate de sodium ; Tf : triflate ; MS : tamis moléculaire ; All : allyle ; PMB : *p*-méthoxybenzyle ; SE : triméthylsilyléthyle. Dowex®, Sephadex®, Chelex®, Toyopearl® sont des marques déposées.

Dans les méthodes, les préparations et dans les exemples décrits ci-après, des modes opératoires généraux concernant le couplage catalytique des imidates, le clivage des esters lévuliniques, le couplage catalytique des thioglycosides, la saponification, la méthylation et la déprotection sélective du groupe p-méthoxybenzyle, la déprotection et la sulfatation des oligo- et des polysaccharides par hydrogénolyse des esters ou des éthers benzyliques, la saponification des esters ou encore les sulfatations peuvent être réalisés en appliquant les méthodes générales ci-après aux intermédiaires appropriés.

Les composés de la présente invention sont synthétisés selon les différentes préparations décrites ci-après.

### PREPARATION 1

### 6-O-Tert-butyldiméthylsilyl-1,2-O-isopropylidène-3-O-méthyl-α-D-glucofuranose (2)

On reprend le diol **1** (10 g, 42,7 mmol) dans du dichlorométhane anhydre (100 ml) et on ajoute du chlorure de *tert*-butyldiméthylsilyle (7,1 g, 47,3 mmol) et de l'imidazole (5,8 g, 85,3 mmol). Le mélange réactionnel est agité à température ambiante. Après 2 heures, le mélange est dilué dans du dichlorométhane et lavé à l'eau. La phase organique est séchée sur sulfate de magnésium, concentrée et le résidu est purifié par chromatographie sur une colonne de gel de silice (acétate d'éthyle/cyclohexane 1/9 v/v) afin d'obtenir le produit désiré **2** (11,9 g, 80 %) sous forme d'un sirop.
[α]_{D} - 34°C (*c* 1,9, CHCl₃).

### PREPARATION 2

### 6-O-Tert-butyldiméthylsilyl-1,2-O-isopropylidène-3-O-méthyl-5-C-vinyl-α-D-glucofuranose (4)

On ajoute à - 78°C dans du dichlorométhane anhydre (40 ml) du chlorure d'oxalyle (3,2 ml, 36,8 mmol) et du diméthylsulfoxyde (5,2 ml, 73,4 mmol) et on agite pendant 30 minutes. Ensuite, le composé **2** (6,4 g, 18,4 mmol) est ajouté et on agite le mélange pendant encore 1 heure. Puis, on ajoute de la triéthylamine (15,3 ml, 110,0 mmol) et après 30 minutes, le mélange réactionnel est dilué dans du dichlorométhane. Un traitement classique permet d'obtenir le composé 5-ulose (**3**) qui est directement utilisé pour la réaction suivante. La cétone **3** brut est reprise dans du tétrahydrofurane anhydre (100 ml) et on ajoute une solution 1M de bromure de vinyl magnésium dans le tétrahydrofurane (28 ml, 27,6 mmol) à 0°C. Après 1 heure, le mélange réactionnel est dilué non pas avec du chlorure d'ammonium et lavé à l'eau.

La phase organique est séchée sur sulfate de magnésium, concentrée et le résidu est purifié par chromatographie sur une colonne de gel de silice (acétate d'éthyle/cyclohexane 1/9 v/v) afin d'obtenir le composé désiré **4** (70 %, 4,8 g) sous forme d'un sirop.
[α]_{D} - 40°C (c 1,3, CHCl₃).
Anal. Calculée : C, 57,72, H, 9,15. Trouvé : C, 57,77, H, 9,23.

### PREPARATION 3

### 1,2,4,6-Tetra-O-acétyl-3-O-méthyl-5-C-vinyl-β-D-glucopyranose (6)

Le composé **4** (3,5 g, 9,4 mmol) est repris dans l'eau (50 ml) ; on y ajoute de la résine IR-120 (1 g) et on chauffe à 80°C pendant 6 heures. La résine est filtrée et le filtrat est concentré. Le produit brut 5 est acétylé en utilisant de l'anhydride acétique (12 ml) et de la pyridine (13 ml). L'excès d'anhydride acétique est détruit avec du méthanol et les solvants sont concentrés. Le résidu est extrait avec de l'eau et du dichlorométhane. La phase organique est séchée sur sulfate de magnésium, concentrée et après purification par chromatographie sur une colonne de gel de silice (acétate d'éthyle/ cyclohexane 3/2 v/v), le composé tétra-acétate 6 est obtenu sous forme d'un solide (75 %, 2,7 g). Pf 50°C.
[α]_{D} - 84°C (c 1,6, CHCl₃).
Anal. Calculée : C, 52,47, H, 6,19. Trouvé : C, 52,51, H, 6,19.
CI-MS : 406 (M + NH₄), 389 (M + 1).

### PREPARATION 4

### Méthyl 2,3,6-tri-O-benzyl-4-O-(2,4,6-tri-O-acétyl-3-O-méthyl-5-C-vinyl-β-D-glucopyranosyl)-α-D-glucopyranoside (8)

Le composé **6** (1,6 g, 4,1 mmol) et le composé **7** (2,1 g, 4,5 mmol) P.J. Garegg and H. Hultberg, Carbohydr. Res. 1981, 93, C10, sont solubilisés dans du dichlorométhane anhydre (50 ml) et on ajoute un tamis moléculaire (4,0 g). Le mélange réactionnel est agité à température ambiante pendant une heure puis on ajoute du TMSOTf (0,95 ml, 5,2 mmol) à - 78°C. On laisse ensuite le mélange réactionnel revenir doucement à la température ambiante. Après 2 heures, le mélange réactionnel est neutralisé avec de la triéthylamine et filtré sur Célite ; le filtrat est lavé à l'eau. La phase organique est séchée sur sulfate de magnésium, concentrée et le résidu est purifié par chromatographie sur gel de silice (acétate d'éthyle/cyclohexane 4/1 v/v) pour obtenir le composé désiré **8** (2,77 g, 85 %) sous forme d'un solide. Pf 47°C.
[α]_{D} - 36°C (c 0,6, CHCl₃).
Anal. Calculée : C, 65,14, H, 6,61. Trouvé : C, 65,09, H, 6,70.

### PREPARATION 5

### Méthyl 2,3,6-O-tri-O-benzyl-4-O-(4,6-O-isopropylidène-3-O-méthyl-5-C-vinyl-β-D-glucopyranosyl)-α-D-glucopyranoside (10)

Le composé **8** (2,7 g, 3,4 mmol) est solubilisé dans du méthanol (40 ml). On ajoute du sodium (catalytique) à 0°C et on agite à température ambiante pendant 3 heures. Le solvant est concentré et le résidu **9** est repris dans de l'acétone anhydre (40 ml) et on ajoute du 2,2-diméthoxypropane (2 ml) et de l'acide p-toluène sulfonique (catalytique). Le mélange réactionnel est agité à température ambiante pendant une nuit. Le solvant est évaporé, le résidu est repris dans du chloroforme et lavé à l'eau. La phase organique est séchée sur sulfate de magnésium, concentrée et le résidu est purifié par chromatographie sur une colonne de silice (acétate d'éthyle/cyclohexane 1/1 v/v) pour obtenir le dérivé 4', 6' isopropylidène -O-10 (1,7 g, 70 %) sous forme d'un solide. Pf 55°C.
[α]_{D} + 13°C (c 0,8, CHCl₃).
Anal. Calculée : C, 67,97, H, 7,13. Trouvé : C, 67,87, H, 7,16.
CI-MS : 707 (M + 1), 724 (M + NH₄).

### PREPARATION 6

### Méthyl 2,3,6-tri-O-benzyl-4-O-(4,6-O-isopropylldène-3-O-méthyl-5-C-vinyl-β-D-mannopyranosyl)-α-D-glucopyranoside (12)

On agite du chlorure d'oxalyle (0,35 ml, 4,0 mmol) et du DMSO anhydre (0,57 ml, 8,0 mmol) dans du dichlorométhane anhydre (10 ml) à -78°C pendant 30 minutes. Le composé **10** (1,4 g, 2,0 mmol) dans du dichlorométhane anhydre (10 ml) est ajouté à la solution et on agite pendant encore 45 minutes. Le mélange réactionnel est neutralisé par addition de triéthylamine anhydre (1,7 ml, 12,0 mmol) puis dilué avec du dichlorométhane. Après l'avoir lavée à l'eau, la phase organique est séchée sur sulfate de magnésium, concentrée et le résidu **11** est directement utilisé pour la réaction suivante sans purification. La cétone **11** est reprise dans du tétrahydrofurane anhydre (15 ml) et on ajoute une solution 1N de super hydrure dans le tétrahydrofurane (4 ml, 4,0 mmol) à - 78°C. Le mélange réactionnel est agité à température ambiante pendant 1 heure et on ajoute ensuite de l'hydroxyde de sodium à 5 % (2 ml) et du peroxyde d'hydrogène (1 ml). Le solvant est évaporé et le résidu est repris par de l'acétate d'éthyle et lavé à l'eau. La phase organique est séchée sur sulfate de magnésium, concentrée et le résidu est purifié par chromatographie (acétate d'éthyle/cyclohexane 2/1 v/v) pour obtenir le composé **12** (1,0 g, 70 %).
[α]_{D} - 11°C (c 0,5, CHCl₃).
CI-MS : 724 (M + 18), 707 (M + 1).

### PREPARATION 7

### Méthyl 2,3,6-tri-O-benzyl-4-O-(2-O-acétyl-3-O-méthyl-5-C-vinyl-β-D-mannopyranosyl)-α-D-glucopyranoside (14)

Le composé **12** (940 mg, 1,3 mmol) est dissous dans la pyridine (3 ml) et on ajoute de l'anhydride acétique (0,3 ml). Le mélange réactionnel est agité à température ambiante pendant 3 heures. L'excès de pyridine et d'anhydride acétique est concentré et le résidu **13** est directement utilisé pour la déprotection de l'isopropylidène en utilisant de l'acide acétique à 80 % (5 ml) à 60°C pendant 2 heures. L'excès d'acide acétique est évaporé et le résidu est purifié par une chromatographie sur une colonne de gel de silice (acétate d'éthyle/cyclohexane 4/1 v/v) pour obtenir le diol **14** (660 mg, 70 %) sous forme solide. Pf 53°C.
[α]_{D} - 10°C (c 0,8, CHCl₃).
CI-MS : 709 (M + 1), 726 (M + 18).

### PREPARATION 8

### Méthyl 2,3,6-tri-O-benzyl-4-O-(2-O-acétyl-3-O-méthyl-6-O-tosyl-5-C-vinyl-β-D-mannopyranosyl)-α-D-glucopyranose (15)

Le composé **14** (600 mg, 0,9 mmol) est dissous dans la pyridine (3 ml) et on ajoute du chlorure de tosyle (240 mg, 1,3 mmol). Le mélange réactionnel est agité à température ambiante pendant 3 heures. Le solvant est évaporé, le résidu est dilué avec du chloroforme et lavé à l'eau. La phase organique est séchée sur sulfate de magnésium, concentrée et le résidu est purifié par chromatographie sur une colonne de gel de silice (acétate d'éthyle/cyclohexane 1/1 v/v) pour obtenir le composé tosylé **15** (297 mg, 80 %) sous forme d'un sirop.
[α]_{D} - 26°C (c 0,8, CHCl₃).

### PREPARATION 9

### Méthyl 2,3,6-tri-O-benzyl-4-O-(2,6-anhydro-3-O-méthyl-5-C-vlnyl-β-D-mannopyranosyl)-α-D-glucopyranoside (16)

Le composé **15** (550 mg, 0,6 mmol) est repris dans l'éthanol (3 ml) et on ajoute ensuite une solution 0,1N d'hydroxyde de sodium éthanolique (5 ml). Le mélange réactionnel est chauffé à 70°C pendant 3 heures puis neutralisé par une résine IR-120 (forme H⁺) et filtré sur Célite. Le résidu, après concentration, est purifié par chromatographie sur une colonne de gel de silice (acétate d'éthyle/cyclohexane 1/1 v/v) pour obtenir le composé **16** (292 mg, 70 %) sous forme d'un sirop.
[α]_{D} + 13°C (c 0,5, CHCl₃).
CI-MS : 666 (M + 18).

### PREPARATION 10

### Méthyl 2,3,6-tri-O-benzyl-4-O-(benzyl 3-O-méthyl-2-O-5-C-méthylidène-α-L-idopyranuronate)-α-D-glucopyranoside (17)

Le composé **16** (260 mg, 0,4 mmol) est dissout dans du dichlorométhane (20 ml), la solution est agitée à - 78° C puis on fait barbotter de l'ozone pendant 30 secondes. La couleur de la solution devient jaune pâle. On ajoute du diméthylsulfure à la solution puis le mélange réactionnel est lavé à l'eau. La phase organique est séchée sur sulfate de magnésium, concentrée et on passe directement à la réaction suivante sans purification supplémentaire. L'aldéhyde brut est repris dans du *tert*-butanol (16 ml) et on ajoute du 2-méthyl-2-butène (5 ml) et de l'eau (16 ml). On ajoute ensuite successivement au mélange du NaH₂PO₄ (700 mg) et du NaClO₂ (700 mg). La suspension est vigoureusement agitée à tempéraure ambiante pendant une nuit, diluée à l'eau et extraite à l'acétate d'étyle. La phase organique est séchée sur sulfate de magnésium, concentrée puis on passe directement à la réaction suivante. L'acide brut est repris dans du diméthylformamide (25 ml) et on ajoute de l'iodure de tétrabutylammonium (0,7 g, 2,0 mmol), du bicarbonate de potassium (0,25 g, 2,5 mmol) et du bromure de benzyle (0,250 ml, 2,1 mmol). Le mélange réactionnel est agité à température ambiante pendant 5 heures. Le mélange réactionnel est extrait avec de l'eau et de l'éther diéthylique. La phase éthérée est séchée sur sulfate de magnésium, concentrée et le résidu est purifié par chromatographie sur une colonne de gel de silice (acétate d'éthyle/cyclohexane 2/1 v/v) pour obtenir le dérivé **17** (236 mg, 80 %) sous forme d'un sirop. CI-MS : 774 (M + 18).

### PREPARATION 11

### Méthyl O-(6-O-acétyl-2,3,4-tri-O-méthyl-α-D-glucopyranosyl)-(1→4)-O-(benzyl 2,3-di-O-méthyl-β-D-glucopyranosyluronate)-(1→4)-O-(3,6-di-O-acétyl-2-O-benzyl-α-D-glucopyranosyl)-(1→4)-O-(2,6-anhydro-5-C-benzyloxycarbonyl-3-O-méthyl-β-D-mannopyranosyl)-(1→4)-2,3,6-tri-O-benzyl-α-D-glucopyranoside (19).

On agite sous atmosphère inerte, l'imidate **18** (Van der Heijden *et al*., abstr. 9^{th} Eur. Carbohydr. Symp., Utrecht, July 6-11, 1997; A74, p 154) (81 mg, 78,2 µmol) l'accepteur **17** (65 mg, 86,0 µmol) et du fin tamis moléculaire (70 mg, 4 Å) dans un mélange dichlorométhane/éther diéthylique, 1/2 (v/v) (2,4 ml).

On agite le mélange réactionnel pendant 30 minutes, puis on ajoute du NaHCO₃ jusqu'à neutralisation. Après filtration et concentration, le résidu est purifié sur une colonne de gel de Sephadex® LH 20 (dichlorométhane/ éthanol 1/1 v/v) puis sur une colonne de gel de silice (acétate d'éthyle/cyclohexane 1/1 (v/v)) pour obtenir le composé **19** (86 mg, 67 %).
[α]_{D} + 66°C (c,1,0,CH₂ CH₂)
¹HRMN présentée dans le TABLEAU 1, ci-après.

### PREPARATION 12

### Méthyl O-(2,3,4-tri-O-méthyl-α-D-glucopyranosyl)-(1→4)-O-(acide 2,3-di-O-méthyl-β-D-glucopyranosyluronique)-(1→4)-O-(α-D-glucopyranosyl)-(1→4)-O-(2,6-anhydro-5-C-carboxy-3-O-méthyl-β-D-mannopyranosyl)-(1→4)-α-D-glucopyranoside (20).

D'après M. PETITOU et C.A.A. van BOECKEL. Progress in the Chemistry of Organic Natural Products, Eds. W. Herz *et al*., Wein, Springer-Verlag, New York, 1992, 143-210.

On agite sous hydrogène (3,5 MPa) pendant 12 heures à 40°C, une solution du composé **19** (49 mg, 30,0 µmol) dans l'acide acétique (3 ml) en présence de palladium sur charbon à 5% (73 mg, 35 b). On filtre sur celite, concentre et codistile avec de l'eau (4 x 5 ml). Le résidu est dissous dans une solution aqueuse de NaOH 1 M (3 ml) et chauffé à 55°C pendant 3 heures. On refroidit et passe la solution au travers d'une colonnne Sephadex® 625 F (170 ml) éluée à l'eau. Les fractions contenant le composé 20 sont passées sur une colonne de résine Dowex H⁺. On concentre pour isoler le composé 20 (25 mg, 86 %).
[α]_{D} + 105°C (c,1,0,H₂O)
¹HRMN présentée dans le TABLEAU 1, ci-après.

**TABLEAU 1**

| Composé | Unité | H-1 | H-2 | H-3 | H-4 | H-5 | H-6 | H-6' | *J*₁₋₂ (Hz) |
|---|---|---|---|---|---|---|---|---|---|
| 19 (CDCl₃) | D | 5,50 | 3,11 | 3,37 | 3,03 | 3,43 | 4,28 | 4,22 | 3,7 |
| | E | 4,14 | 2,93 | 3,31 | 3,92 | 3,83 | - | - | ∼9 |
| | F | 4,98 | 3,45 | 5,41 | 3,63 | 3,94 | 4,58 | 4,19 | 3,6 |
| | G | 5,18 | 3,83 | 3,12 | 4,12 | - | 4,10 | 3,98 | ∼1 |
| | H | 4,58 | 3,51 | 4,01 | 3,78 | 3,80 | 3,83 | 3,61 | 3,6 |
| | | | | | | | | | |
| 20 (D₂O) | D | 5,50 | 3,33 | 3,49 | 3,30 | 3,50 | 3,74 | 3,74 | 3,9 |
| | E | 4,62 | 3,26 | 3,58 | 3,90 | 4,04 | - | - | 7,9 |
| | F | 5,13 | 3,58 | 3,76 | 3,61 | 3,70 to 3,90 | | | 4,1 |
| | G | 5,23 | 4,35 | 3,71 | 4,20 | - | 4,25 | 4,10 | ∼1 |
| | H | 4,82 | 3,64 | 3,84 | 3,73 | 3,70 to 3,90 | | | 3,7 |

### PREPARATION 13

### Méthyl O-(4,6-O-isopropylidène-2,3-di-O-méthyl-5-C-vinyl-β-D-glucopyranosyl)-(1→4)-2,3,6-tri-O-benzyl-α-D-glucopyranoside (22).

On ajoute, à 0° C, de l'hydrure de sodium (0,31 g, 13,0 mmol) à une solution de méthyl *O*-(4,6-*O*-isopropylidène-3-*O*-méthyl-5-C-vinyl-β-D-glucopyranosyl)-(1→4)-2,3,6-tri-*O*-benzyl-α-D-glucopyranoside **(10)** (6,11 g, 8,65 mmol) de l'iodure de méthyle (0,80 ml, 13,0 mmol) dans le *N,N*-diméthylformamide (9,00 ml). On laisse sous agitation pendant 2 heures (CCM), on additionne du méthanol, et on verse le mélange réactionnel dans l'eau. On extrait avec de l'acétate d'éthyle, on lave avec de l'eau, on sèche et on concentre. On purifie sur colonne de silice (cyclohexane/éther diéthylique 3/2 (v/v)) pour obtenir **22** (5,92 g, 88 %). CCM *R*_{f} 0,28, (cyclohexane/éther diéthylique 3/2 (v/v)).

### PREPARATION 14

### Méthyl O-(4,6-O-isopropylidène-2,3-di-O-méthyl-5-C-éthyl-β-D-glucopyranosyl)-(1→4)-2,3,6-tri-O-benzyl-α-D-glucopyranoside (23).

On ajoute de l'oxyde de platine (160 mg) à une solution de **22** (5,80 g, 8,04 mmol) dans de l'acétate d'éthyle (400 ml). On introduit de l'hydrogène. On laisse sous agitation pendant 40 minutes (CCM), on filtre, on évapore pour obtenir **23.** CCM *R*_{f} 0,60, (toluène/acétate d'éthyle 4/1 (v/v)).

### PREPARATION 15

### Méthyl O-(2,3-di-O-méthyl-5-C-éthyl-β-D-glucopyranosyl)-(1→4)-2,3,6-tri-O-benzyl-α-D-glucopyranoside (24).

Le composé brut **23** est dissous dans de l'acide acétique à 70 % (60 ml) et agité pendant 2 heures à 80° C. Le mélange est concentré sous vide et co-évaporé avec du toluène pour donner **24.** CCM *R*_{f} 0,52 , (dichlorométhane/ méthanol 4/1 (v/v)).

### PREPARATION 16

### Méthyl O-(benzyl 2,3-di-O-méthyl-5-C-éthyl-β-D-glucopyranosyluronate)-(1→4)-2,3,6-tri-O-benzyl-α-D-glucopyranoside (25).

À une solution du composé **24** (5,75 g) dans du tétrahydrofurane (28 ml), on ajoute du 2,2,6,6-tétraméthyl-1-pipéridinyloxy (17,5 mg), une solution d'hydrogénocarbonate de sodium (17,5 ml), du bromure de potassium (87 mg) et du chlorure de tétrabutylammonium (115,5 mg). Le mélange est refroidi à 0°C et on ajoute pendant 15 minutes un mélange d'une solution saturée de chlorure de sodium (17 ml), d'une solution saturée d'hydrogénocarbonate de sodium (8,70 ml) et d'hypochlorite de sodium (1,3 M, 20 ml). Après agitation pendant 1 heure, le mélange est dilué avec de l'eau et extrait (3 fois), avec du dichlorométhane. La phase organique est lavée avec une solution aqueuse de chlorure de sodium, séchée sur sulfate de magnésium, filtrée et évaporée à sec pour donner le dérivé acide brut.

Sous atmosphère d'azote, le dérivé acide est dissous dans du *N,N*-diméthylformamide (107 ml). On ajoute de l'hydrogénocarbonate de potassium (4,10 g) et du bromure de benzyle (9,70 ml) et le mélange est agité pendant 16 heures. On ajoute de l'acétate d'éthyle et de l'eau puis après extraction, la phase organique est concentrée. Une purification par chromatographie sur colonne de gel de silice permet d'obtenir 3,81 g du composé 25 (60 % de rendement à partir du composé **23).**
[α]_{D} + 24 (c = 0,15, dichlorométhane).

### PREPARATION 17

### Méthyl O-(benzyl 5-C-éthyl-4-O-lévulinyl-2,3-di-O-méthyl-β-D-glucopyranosyluronate)-(1→4)-2,3,6-tri-O-benzyl-α-D-glucopyranoside (26).

On dissout le composé **25** (3,51 g, 4,46 mmol) dans du dioxane anhydre (45 ml). On ajoute de l'acide lévulinique (1,00 g, 8,93 mmol), du chlorhydrate de 1-(3-diméthylaminopropyl)-3-éthylcarbodiimide (1,70 g, 8,93 mmol) et de la 4-diméthylaminopyridine (0,11 g, 8,93 mmol). On laisse sous agitation pendant 16 heures, on extrait avec de l'acétate d'éthyle, on lave successivement avec une solution aqueuse à 5 % d'hydrogénosulfate de potassium, à l'eau, avec une solution aqueuse saturée d'hydrogénocarbonate de sodium, à l'eau, on sèche et on concentre.

On purifie sur colonne de silice (cyclohexane/acétate d'éthyle 2/1 puis 3/2 (v/v)) pour obtenir **26** pur (3,64 g, 85 %). [α]_{D} + 26 (c = 0,9, dichlorométhane).

### PREPARATION 18

### O-(Benzyl 5-C-éthyl-4-O-lévulinyl-2,3-di-O-méthyl-β-D-glucopyranosyluronate)-(1→4)-1,3,6-tri-O-acétyl-2-O-benzyl-D-glucopyranose (27).

On dissout le composé **26** (3,35 g, 3,78 mmol) dans l'anhydride acétique (22 ml). On refroidit à - 20 °C, on ajoute 22 ml d'une solution froide d'acide sulfurique dans l'anhydride acétique (1 ml d'acide sulfurique dans 10 ml d'anhydride acétique).

On dilue avec de l'acétate d'éthyle, on lave avec une solution aqueuse saturée d'hydrogénocarbonate de sodium, à l'eau, on sèche, on concentre. On purifie sur colonne de silice (cyclohexane/acétate d'éthyle 1/1 (v/v)) pour obtenir **27** (2,20 g, 65,5 %). CCM *R*_{f} 0,24, (cyclohexane/acétate d'éthyle 1/1 (v/v)).

### PREPARATION 19

### O-(Benzyl 5-C-éthyl-4-O-lévulinyl-2,3-di-O-méthyl-β-D-glucopyranosyluronate)-(1→4)-3,6-di-O-acétyl-2-O-benzyl-D-glucopyranose (28).

On ajoute de la benzylamine (11 ml, 101,4 mmol) à une solution du composé **27** (2,18 g, 2,67 mmol) dans du tétrahydrofurane (50 ml). On laisse sous agitation pendant 4 heures. On extrait avec de l'acétate d'éthyle, on lave avec une solution aqueuse 1M d'acide chlorhydrique (102 ml), à l'eau, on sèche et on concentre. On purifie sur colonne de silice (toluène/acétate d'éthyle 1/ 1 (v/v)) pour obtenir un mélange (α/β = 50/50) du composé **28** (1,33 g, 65 %). CCM *R*_{f} 0,22 , (toluène/acétate d'éthyle 1/1 (v/v)).

### PREPARATION 20

### O-(Benzyl 5-C-éthyl-4-O-lévulinyl-2,3-di-O-méthyl-β-D-glucopyranosyluronate)-(1→4)-3,6-di-O-acétyl-2-O-benzyl-D-glucopyranosyl trichloroacétimidate (29).

On dissout le composé **28** (1,32 g, 1,71 mmol) dans du dichlorométhane (34 ml) et on ajoute sous argon du trichloroacétonitrile (0,87 ml, 8,50 mmol) et du carbonate de césium (0,90 g, 2,73 mmol). On laisse sous agitation pendant 2 heures (CCM) et on filtre. On purifie sur colonne de silice (cyclohexane/acétate d'éthyle 3/2 (v/v)) pour obtenir un mélange (α/β = 85/15) d'imidates **29** (1,20 g, 77%). CCM *R*_{f} 0,36, (cyclohexane/acétate d'éthyle 1/2 (v/v)).

### PREPARATION 21

### Méthyl O-(benzyl 5-C-éthyl-4-O-lévulinyl-2,3-di-O-méthyl-β-D-glucopyranosyluronate)-(1→4)-O-(3,6-di-O-acétyl-2-O-benzyl-α-D-glucopyranosyl)-(1→4)-O-(2,6-anhydro-5-C-benzyloxycarbonyl-3-O-méthyl-β-D-mannopyranosyl)-(1→4)-2,3,6-tri-O-benzyl-α-D-glucopyranoside (30).

Une solution de triflate de *tert*-butyldiméthylsilyle dans du dichlorométhane (1M, 0,19 ml) est ajoutée, sous argon à - 20° C, à une solution de l'imidate **29** (1,19 g, 1,29 mmol) et de méthyl 2,3,6-tri-*O*-benzyl-4-(benzyl 3-*O*-méthyl-2-*O*-5-*C*-méthylidène-α-L-idopyranuronate)-α-D-glucopyranoside 17 (1,02 g, 1,35 mmol) dans le toluène (40 ml) en présence de tamis moléculaire 4 Å (1,93 g). Après 30 minutes (CCM), on ajoute de nouveau une solution de triflate de *tert*-butyldiméthylsilyle dans du dichlorométhane (1M, 0,19 ml). Après 30 minutes (CCM), on ajoute de l'hydrogénocarbonate de sodium solide. On filtre la solution, on lave à l'eau, on sèche et on évapore à sec. On purifie sur colonne de chromatographie Sephadex® LH20 puis sur colonne de silice (toluène/acétate d'éthyle 2/1 (v/v)) pour obtenir le tétrasaccharide **30**-α pur (1,14 g, 58 %). [α]_{D} + 47 (c = 0,21, dichlorométhane).

### PREPARATION 22

### Méthyl O-(benzyl 5-C-éthyl-2,3-di-O-méthyl-β-D-glucopyranosyluronate)-(1→4)-O-(3,6-di-O-acétyl-2-O-benzyl-α-D-glucopyranosyl)-(1→4)-O-(2,6-anhydro-5-C-benzyloxycarbonyl-3-O-méthyl-β-D-mannopyranosyl)-(1→4)-2,3,6-tri-O-benzyl-a-D-glucopyranoside (31).

On dissout le composé **30** (1,13 g, 0,75 mmol) dans un mélange éthanol/toluène 2/1 (v/v) (150 ml) et on ajoute l'acétate d'hydrazine (0,35 g, 3,73 mmol). On laisse agiter pendant 1 heure (CCM) et on concentre. On purifie sur colonne de silice (toluène/acétate d'éthyle 3/2 (v/v)) pour obtenir **31** (0.816 g, 83 %).
[α]_{D} + 35 (c = 1,01, dichlorométhane).

### PREPARATION 23

### Méthyl O-(6-O-acétyl-2,3,4-tri-O-méthyl-α-D-glucopyranosyl)-(1→4)-O-(benzyl 5-C-éthyl-2,3-di-O-méthyl-β-D-glucopyranosyluronate)-(1→4)-O-(3,6-di-O-acétyl-2-O-benzyl-α-D-glucopyranosyl)-(1→4)-O-(2,6-anhydro-5-C-benzyloxycarbonyl-3-O-méthyl-β-D-mannopyranosyl)-(1→4)-2,3,6-tri-O-benzyl-α-D-glucopyranoside (33).

On traite le trichloroacétimidate de 6-*O*-acétyl-2,3,4-tri-*O*-méthyl-D-glucopyranose **32** (34,7 mg, 0,0245 mmol) (P. Westerduin, *et al*. BioOrg. Med. Chem., 1994, *2*, 1267) et l'accepteur de glycosyle **31** (80 mg, 0,056 mmol) selon la PREPARATION 21 . On purifie le composé sur une colonne de chromatographie Séphadex® LH-20 (dichlorométhane/éthanol 1/1 (v/v)), puis sur une colonne de silice (diisopropyl éther/acétate d'éthyle 3/2 (v/v)) pour obtenir le dérivé **33** (54,6 mg, 58 %).
[α]_{D} + 55 (c = 1, dichlorométhane).

### PREPARATION 24

### Méthyl O-(6-O-acétyl-2,3,4-tri-O-méthyl-α-D-glucopyranosyl)-(1→4)-O-(acide 5-C-éthyl-2,3-di-O-méthyl-β-D-glucopyranosyluronique)-(1→4)-O-(3,6-di-O-acétyl-α-D-glucopyranosyl)-(1→4)-O-(2,6-anhydro-5-C-carboxy-3-O-méthyl-β-D-mannopyranosyl)-(1→4)-α-D-glucopyranoside (34).

Une solution du composé **33** (40 mg, 0,024 mmol) dans de l'acide acétique(2 ml) est agitée sous atmosphère d'hydrogène en présence de palladium sur charbon 10% (80 mg) pendant 16 heures et filtrée. Le filtrat est concentré pour donner le composé **34.**

### PREPARATION 25

### Méthyl O-(2,3,4-tri-O-méthyl-α-D-glucopyranosyl)-(1→4)-O-(acide 5-C-éthyl-2,3-di-O-méthyl-β-D-glucopyranosyluronique)-(1→4)-O-(α-D-glucopyranosyl)-(1→4)-O-(2,6-anhydro-5-C-carboxy-3-O-méthyl-β-D-mannopyranosyl)-(1→4)-α-D-glucopyranoside (35).

On ajoute une solution aqueuse d'hydroxyde de sodium 5M (216 µl) à une solution du composé brut **34** dans le méthanol (866 µl). Après 25 heures on introduit de l'eau et on passe le mélange réactionnel au travers d'une colonne de gel Sephadex® G-25 (2 x 38 cm) éluée par l'eau. On concentre, on passe au travers d'une d'une colonne Dowex® 50 H⁺(2 ml) et on lyophilise. À ce stade on vérifie par ¹H RMN que tous les groupes protecteurs ont été enlevés.

### EXEMPLE 1

### Méthyl O-(2,3,4-tri-O-méthyl-6-O-sulfo-α-D-glucopyranosyl)-(1→4)-O-(acide 2,3-di-O-méthyl-β-D-glucopyranosyluronique)-(1→4)-O-(2,3,4-tri-O-sulfo-α-D-glucopyranosyl)-(1→4)-O-(2,6-anhydro-5-C-carboxy-3-O-méthyl-β-D-mannopyranosyl)-(1→4)-2,3,6-tri-O-sulfo-α-D-glucopyranoside, sel de sodium (21).

D'après C.A.A. van BOECKEL et M. PETITOU, Angew. Chem. Int. Ed. Engl., 1993, *32*, 1671-1690.

Une solution du composé **20** (20 mg, 20,7 µmol) et du complexe triéthylamine/trioxyde de soufre (164 mg, 0,90 µmol) dans le diméthylformamide (2 ml) est chauffée à 55°C à l'abri de la lumière pendant 18 heures et 30 minutes. On refroidit le mélange réactionnel à température ambiante puis dilue avec une solution aqueuse de NaCI 0,2 M. On dépose alors la solution au sommet d'une colonne de Sephadex® G25F (170 ml) éluée par une solution aqueuse de NaCl 0,2 M.

On concentre les fractions contenant le pentasaccharide et on dessale en utilisant la même colonne éluée par l'eau. Après lyophilisation, le composé **21** est obtenu (30,5 mg, 85 %)
[α]_{D} + 49°C (c,0,63,H₂O)
¹HRMN présentée dans le TABLEAU 2 ci-après.

**TABLEAU 2**

| Composé | Unité | H-1 | H-2 | H-3 | H-4 | H-5 | H-6 | H-6' | *J*₁₋₂ (Hz) |
|---|---|---|---|---|---|---|---|---|---|
| 21 (D₂O) | D | 5,46 | 3,32 | 3,55 | 3,34 | 3,87 | 4,28 | 4,12 | 3,9 |
| | E | 4,66 | 3,26 | 3,53 | 3,89 | 3,73 | - | - | 7,9 |
| | F | 5,50 | 4,36 | 4,81 | 4,00 | 4,17 | 4,49 | 4,41 | 3,7 |
| | G | 5,49 | 4,41 | 3,73 | 4,17 | - | 4,24 | 4,09 | -1 |
| | H | 5,16 | 4,36 | 4,52 | 4,01 | 4,08 | 4,41 | 4,30 | 3,7 |

### EXEMPLE 2 Méthyl O-(2,3,4-tri-O-méthyl-6-O-sulfo-α-D-glucopyranosyl)-(1→4)-O-(acide 5-C-éthyl-2,3-di-O-méthyl-β-D-glucopyranosyluronlque)-(1→4)-O-(2,3,6-tri-O-sulfo-α-D-glucopyranosyl)-(1→4)-O-(2,6-anhydro-5-C-carboxy-3-O-méthyl-β-D-mannopyranosyl)-(1→4)-2,3,6-tri-O-sulfo-α-D-glucopyranoside, sel de sodium (36).

On ajoute du complexe triéthylamine/trioxyde de soufre (91 mg) à une solution dans le diméthylformamide (1,3 ml) du composé brut **35.** Après 20 heures à 55° C on dépose la solution au sommet d'une colonne de Sephadex® G-25 (2 x 38 cm) éluée par du chlorure de sodium 0,2 M. On concentre les fractions contenant le produit et on dessale en utilisant la même colonne éluée par l'eau. Le composé **36** est obtenu après lyophilisation (21,9 mg, 52 % à partir du composé **33**).

¹HRMN présentée dans le TABLEAU 3 ci-après.

**TABLEAU 3**

| | H-1 (J₁₋₂ Hz) | H-2 | H-3 | H-4 | H-5 | H-6 | H-6' | autres |
|---|---|---|---|---|---|---|---|---|
| D | 5,43 (3,9) | 3,28 | 3,53 | 3,30 | 4,02 | 4,27 | 4,14 | |
| E | 4,68 (8,1) | 3,27 | 3,60 | 3,98 | - | - | - | 2,04/1,80: C*H*₂CH₃ 09,4 : CH₂C*H*₃ |
| F | 5,50 (3,7) | 4,35 | 4,50 | 3,84 | 4,07 | 4,61 | 4,30 | |
| G | 5,47 (1,1) | 4,39 | 3,72 | 4,16 | - | 4,23 | 4,08 | |
| H | 5,16 (3,7) | 4,36 | 4,81 | 4,00 | 4,17 | 4,50 | 4,39 | |

En procédant comme pour les exemples 1 et 2 précédents, on prépare les composés 37 à 40 des exemples 3 à 6 suivants. Les spectres RMN ¹H obtenus pour ces composés sont en accord avec les configurations indiquées ci-dessous.

### EXEMPLE 3

### Méthyl O-(2,3,4-tri-O-methyl-6-O-sulfo-α-D-glucopyranosyl)-(1→4)-O-(acide 2,3-di-O-méthyl-β-Dglucopyranosyluronique)-(1→4)-O-(2,3,6-tri-O-sulfo-α-D-glucopyranosyl)-(1→4)-O-(2,6-anhydro-5-C-carboxy-3-O-méthyl-β-D-mannopyranosyl)-(1→4)-2-O-sulfo-3,6-di-O-méthyl-α-D-glucopyranoside, sel de sodium (37).

[α]_{D} +51° (*c* 0,48, eau)

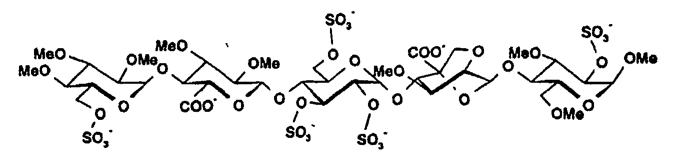

### EXEMPLE 4

### Méthyl O-(2,3,4-tri-O-méthyl-6-O-sulfo-α-D-glucopyranosyl)-(1→4)-O-(acide 2,3-di-O-méthyl-β-Dglucopyranosyluronique)-(1→4)-O-(2,3,6-tri-O-sulfo-α-D-glucopyranosyl)-(1→4)-O-(2,6-anhydro-5-C-carboxy-3-O-méthyl-β-D-mannopyranosyl)-(1→4)-2,3-di-O-sulfo-6-O-méthyl-α-D-glucopyranoside, sel de sodium (38).

[α]_{D} +57° (*c* 0.28. eau)

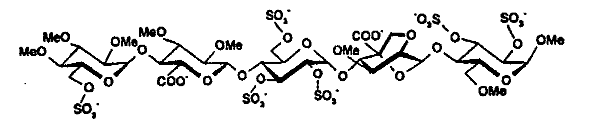

### EXEMPLE 5

### Méthyl O-(2,3,4-tri-O-méthyl-6-O-sulfo-α-D-glucopyranosyl)-(1→4)-O-(acide 2,3-di-O-méthyl-β-Dglucopyranosyluronique)-(1→4)-O-(2,3,6-tri-O-sulfo-α-D-glucopyranosyl)-(1→4)-O-(2,6-anhydro-5-C-carboxy-3-O-méthyl-β-D-mannopyranosyl)-(1→4)- 2,6-di-O-sulfo-3-O-méthyl-α-D-glucopyranoside, sei de sodium ( 39).

[α]_{D} +53° (*c* 0,3, eau)

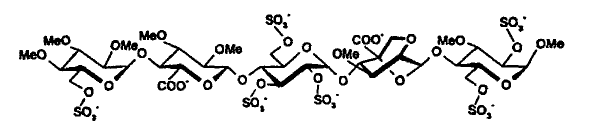

### EXEMPLE 6

### Méthyl O-(2,3,4-tri-O-méthyl-6-O-sulfo-α-D-glucopyranosyl)-(1→4)-O-(acide 2,3-di-O-méthyl-β-Dglucopyranosyluronique)-(1→4)-O-(2,3,6-tri-O-sulfo-α-D-glucopyranosyl)-(1→4)-O-(2,7-anhydro-5-C-carboxy-6-déoxy-3-O-méthyl-β-D-mannoheptopyranosyl)-(1→4)-2,3,6-tri-O-sulfo-α-D-glucopyranoside, sel de sodium (40).

[α]_{D} 49° *(c* 0,25, eau)

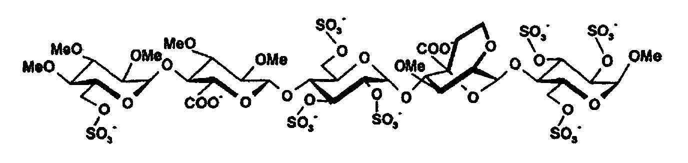

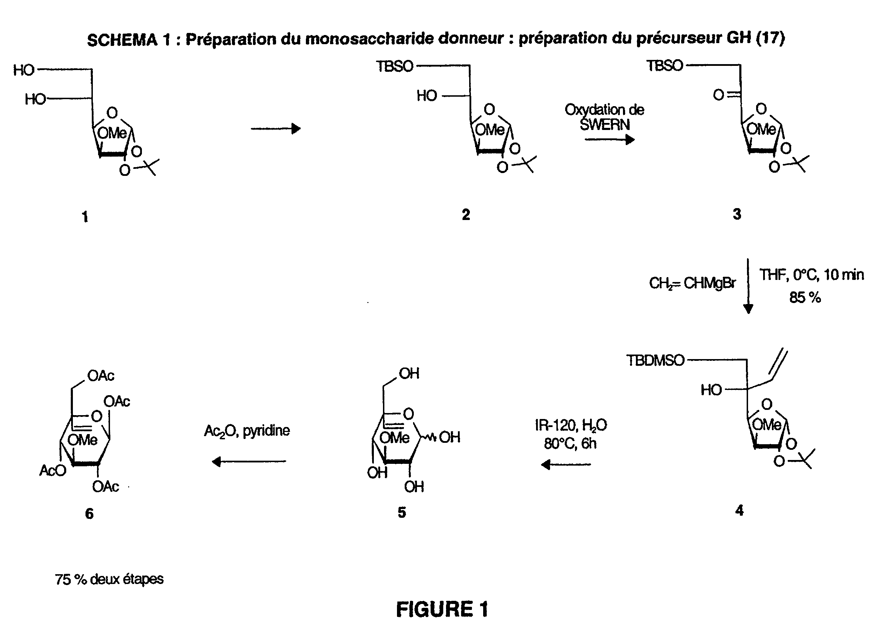

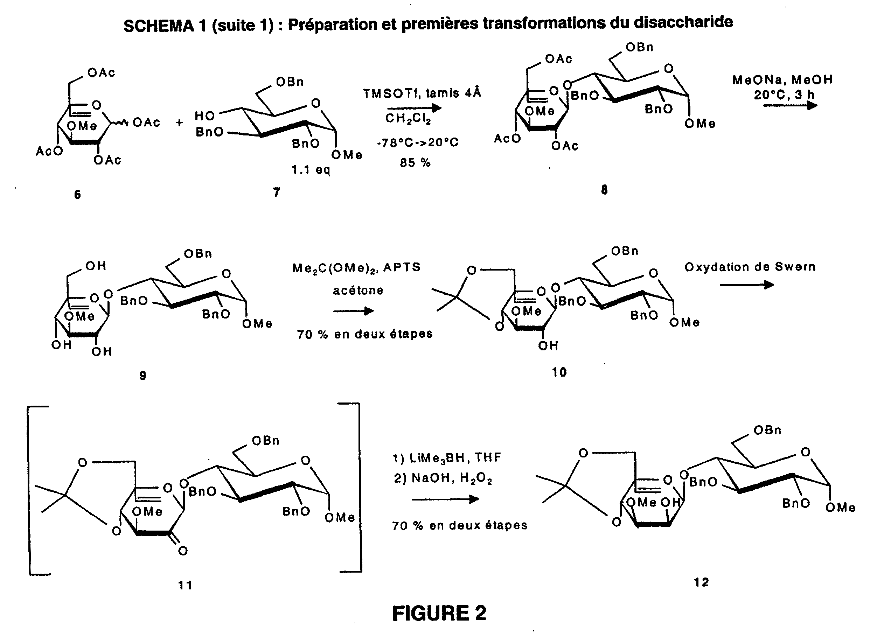

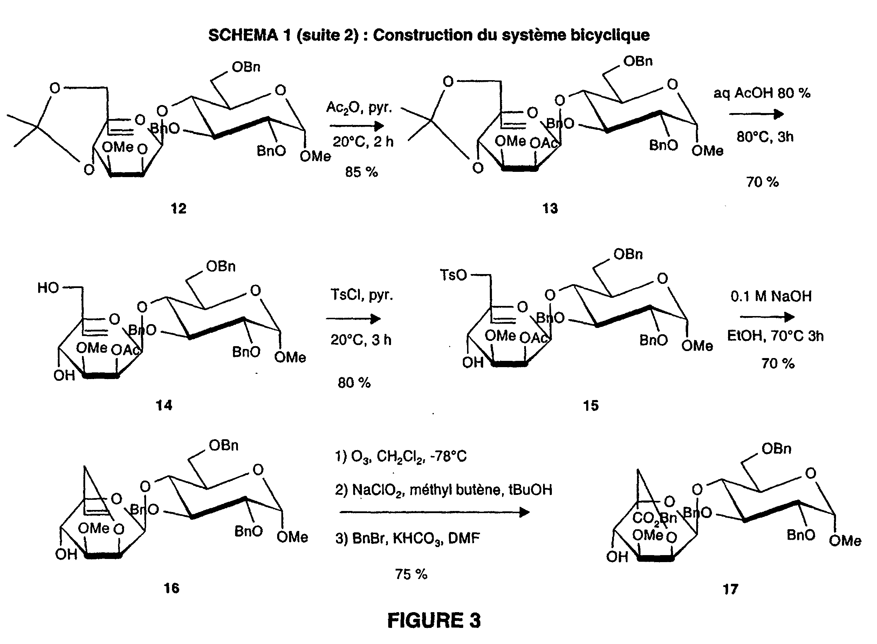

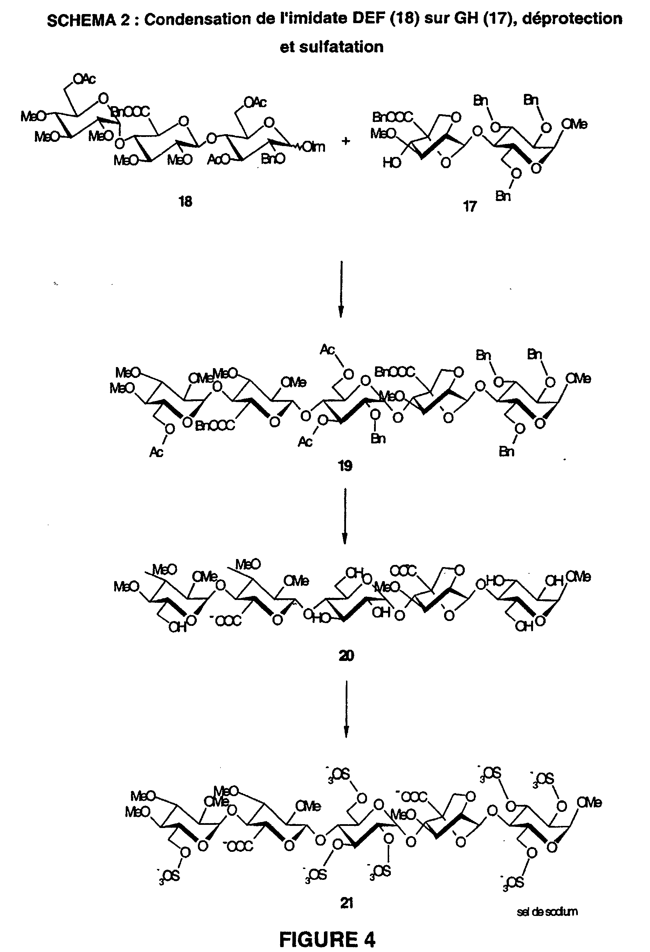

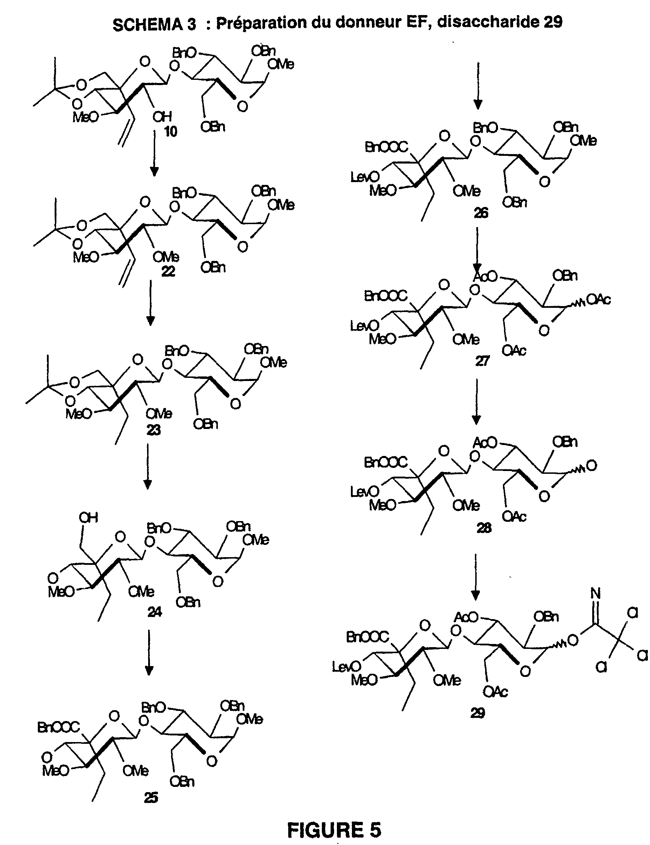

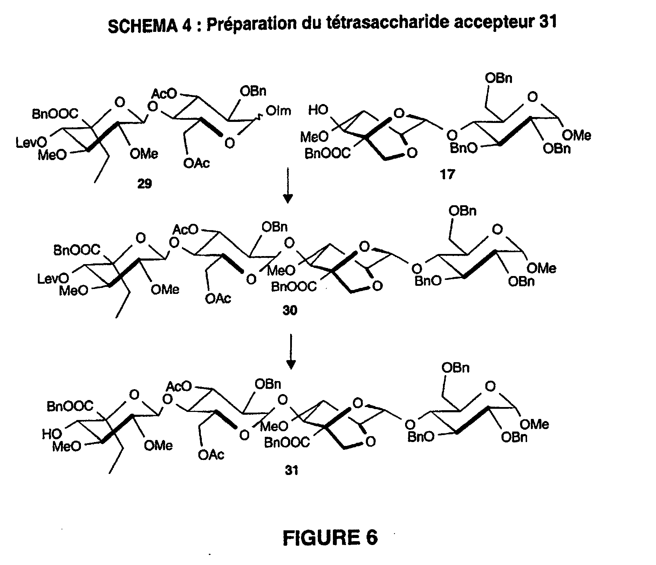

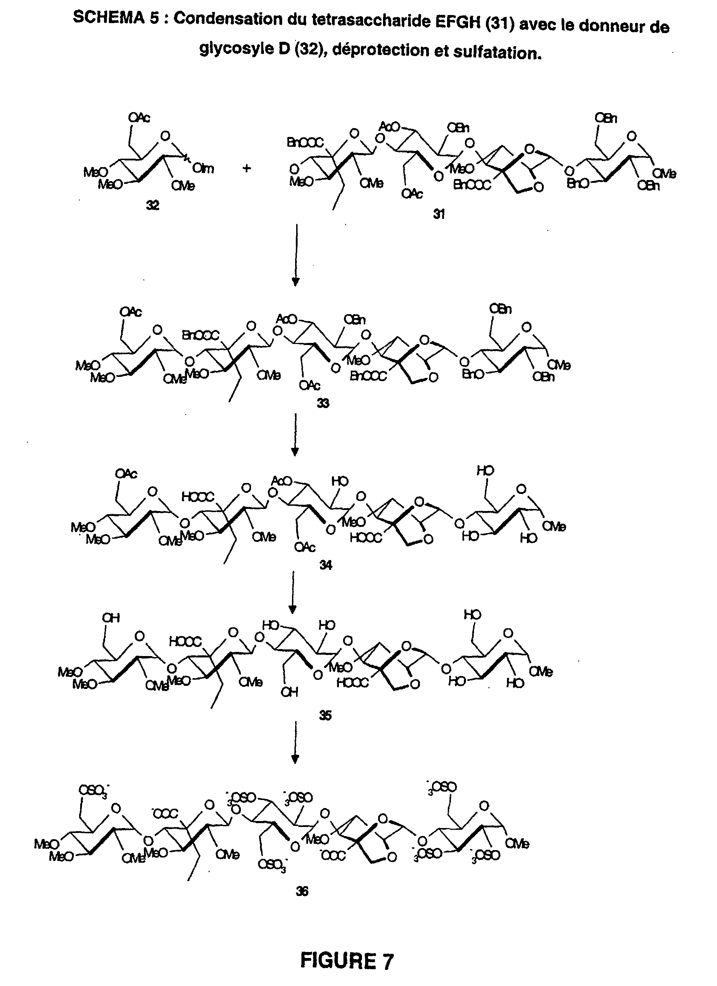

## Revendications

1. Pentasaccharide sous forme acide et ses sels pharmaceutiquement acceptables, dont la forme anionique a pour formule : 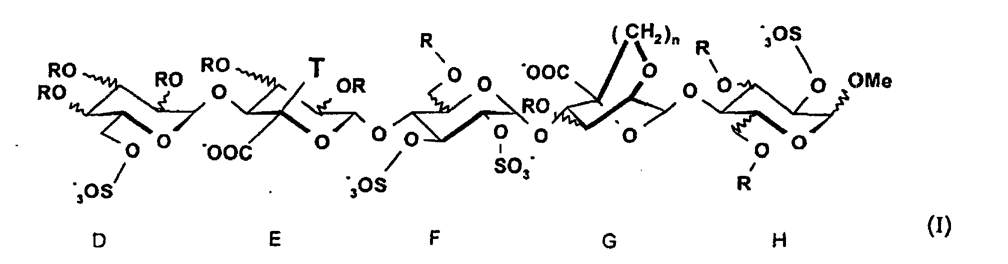 dans laquelle :
- R représente l'hydrogène, un groupe -SO₃⁻, (C₁-C₃) alkyle, ou (C₂-C₃) acyle ;
- T représente l'hydrogène ou un groupe éthyle ;
- n représente 1 ou 2.

2. Pentasaccharide selon la revendication 1 sous la forme de sel de sodium ou de potassium.

3. Pentasaccharide selon l'une quelconque des revendications 1 à 2, choisi parmi:
- Méthyl O-(2,3,4-tri-O-méthyl-6-O-sulfo-α-D-glucopyranosyl)-(1→4)-O-(acide 2,3-di-O-méthyl-β-D-glucopyranosyluronique)-(1→4)-O-(2,3,4-tri-O-sulfo-α-D-glucopyranosyl)-(1→4)-O-(2,6-anhydro-5-C-carboxy-3-O-méthyl-β-D-manno-pyranosyl)-(1→4)-2,3,6-tri-O-sulfo-α-D-glucopyranoside, sel de sodium ;
- Méthyl O-(2,3,4-tri-O-méthyl-6-O-sulfo-α-D-glucopyranosyl)-(1→4)-O-(acide 5-C-éthyl-2,3-di-O-méthyl-β-D-glucopyranosyluronique)-(1→4)-O-(2,3,6-tri-O-sulfo-α-D-glucopyranosyl)-(1→4)-O-(2,6-anhydro-5-C-carboxy-3-O-méthyl-β-D-mannopyranosyl)-(1→4)-2,3,6-tri-O-sulfo-a-D-glucopyranoside, sel de sodium ;
- Méthyl *O*-(2,3,4-tri-O-methyl-6-O-sulfo-α-D-glucopyranosyl)-(1→4)-O-(acide 2,3-di-O-méthyl-β-Dglucopyranosyluronique)-(1→4)-O-(2,3,6-tri-O-sulfo-α-D-glucopyranosyl)-(1→4)-O-(2,6-anhydro-5-C-carboxy-3-O-méthyl-β-D-mannopyranosyl)-(1→4)-2-O-sulfo-3,6-di-O-méthyl-α-D-glucopyranoside, sel de sodium ;
- Méthyl O-(2,3,4-tri-O-méthyl-6-O-sulfo-α-D-glucopyranosyl)-(1→4)-O-(acide 2,3-di-O-méthyl-β-Dglucopyranosyluronique)-(1→4)-O-(2,3,6-tri-O-sulfo-α-D-glucopyranosyl)-(1→4)-O-(2,6-anhydro-5-C-carboxy-3-O-méthyl-β-D-mannopyranosyl)-(1→4)-2,3-di-O-sulfo-6-O-méthyl-α-D-glucopyranoside, sel de sodium ;
- Méthyl O-(2,3,4-tri-O-méthyl-6-O-sulfo-α-D-glucopyranosyl)-(1→4)-O-(acide 2,3-di-O-méthyl-β-Dglucopyranosyluronique)-(1→4)-O-(2,3,6-tri-O-sulfo-α-D-glucopyranosyl)-(1→4)-O-(2,6-anhydro-5-C-carboxy-3-O-méthyl-β-D-mannopyranosyl)-(1→4)- 2,6-di-O-sulfo-3-O-méthyl-α-D-glucopyranoside, sel de sodium ;
- Méthyl O-(2,3,4-tri-O-méthyl-6-O-sulfo-α-D-glucopyranosyl)-(1→4)-O-(acide 2,3-di-O-méthyl-β-Dglucopyranosyluronique)-(1→4)-O-(2,3,6-tri-O-sulfo-α-D-glucopyranosyl)-(1→4)-O-(2,7-anhydro-5-C-carboxy-6-déoxy-3-O-méthyl-β-D-mannoheptopyranosyl)-(1→4)-2,3,6-tri-O-sulfo-a-D-glucopyranoside, sel de sodium.

4. Compositions pharmaceutiques contenant comme principe actif un pentasaccharide selon l'une quelconque des revendications 1 à 3 sous forme de sel avec une base pharmaceutiquement acceptable ou sous forme acide, en association ou en mélange avec un excipient inerte, non toxique, pharmaceutiquement acceptable.

5. Composition pharmaceutique selon la revendication 4, sous forme d'unités de dosage, dans laquelle le principe actif est mélangé à au moins un excipient pharmaceutique.

6. Composition selon la revendication 5 dans laquelle chaque unité de dosage contient de 0,1 à 100 mg de principe actif.

7. Composition selon la revendication 6 dans laquelle chaque unité de dosage contient de 0,5 à 50 mg de principe actif.

8. Utilisation du polysaccharide selon les revendications 1 à 3 pour la préparation d'un médicament utile dans les pathologies dépendantes d'un dysfonctionnement de la coagulation.

## Patentansprüche

1. Pentasaccharid in Form der Säure und seiner pharmazeutisch annehmbaren Salze, deren anionische Form die folgende Formel besitzt: 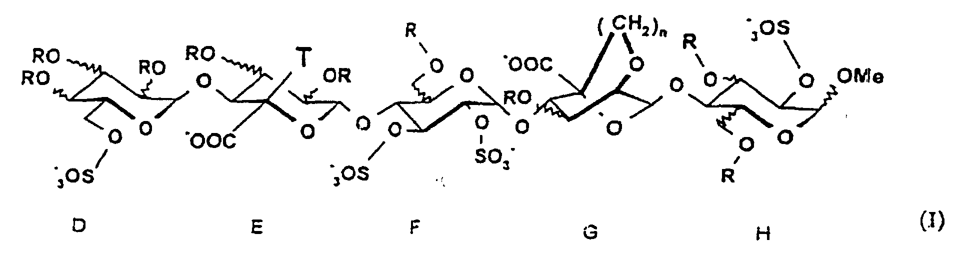 in der:
- R Wasserstoff, eine Gruppe -SO₃⁻, (C₁-C₃)-Alkyl oder (C₂-C₃)-Acyl;
- T Wasserstoff oder eine Ethylgruppe; und
- n 1 oder 2 bedeuten.

2. Pentasaccharid nach Anspruch 1 in Form des Natrium- oder Kaliumsalzes.

3. Pentasaccharid nach irgendeinem der Ansprüche 1 bis 2, ausgewählt aus:
- Methyl-O-(2,3,4-tri-O-methyl-6-O-sulfo-α-D-glucopyranosyl)-(1→4)-O-(2,3-di-O-methyl-β-D-glucopyranosyluronsäure)-(1→4)-O-(2,3,4-tri-O-sulfo-α-D-glucopyranosyl)-(1→4)-O-(2,6-anhydro-5-C-carboxy-3-O-methyl-β-D-mannopyranosyl)-(1→4)-2,3,6-tri-O-sulfo-α-D-glucopyranosid-Natriumsalz;
- Methyl-O-(2,3,4-tri-O-methyl-6-O-sulfo-α-D-glucopyranosyl)-(1→4)-O-(5-C-ethyl-2,3-di-O-methyl-β-D-glucopyranosyluronsäure)-(1→4)-O-(2,3,6-tri-O-sulfo-α-D-glucopyranosyl)-(1→4)-O-(2,6-anhydro-5-C-carboxy-3-O-methyl-β-Dmannopyranosyl)-(1→4)-2,3,6-tri-O-sulfo-α-D-glucopyranosid-Natriumsalz:
- Methyl-O-(2,3,4-tri-O-methyl-6-O-sulfo-α-D-glucopyranosyl)-(1→4)-O-(2,3-di-O-methyl-β-D-glucopyranosyluronsäure)-(1→4)-O-(2,3,6-tri-O-sulfo-α-Dglucopyranosyl)-(1→4)-O-(2,6-anhydro-5-C-carboxy-3-*O*-methyl-β-D-mannopyranosyl)-(1→4)-2-O-sulfo-3,6-di-O-methyl-α-D-glucopyranosid-Natriumsalz:
- Methyl-O-(2,3,4-tri-O-methyl-6-O-sulfo-α-D-glucopyranosyl)-(1→4)-O-(2,3-di-O-methyl-β-D-glucopyranosyluronsäure)-(1→4)-O-(2,3,6-tri-O-sulfo-α-Dglucopyranosyl)-(1→4)-O-(2,6-anhydro-5-C-carboxy-3-O-methyl-β-D-mannopyranosyl)-(1→4)-2,3-di-O-sulfo-6-O-methyl-α-D-glucopyranosid-Natriumsalz;
- Methyl-O-(2,3,4-tri-O-methyl-6-O-sulfo-α-D-glucopyranosyl)-(1→4)-O-(2,3-di-O-methyl-β-D-glucopyranosyluronsäure)-(1→4)-O-(2,3,6-tri-O-sulfo-α-Dglucopyranosyl)-(1→4)-O-(2,6-anhydro-5-C-carboxy-3-O-methyl-β-D-mannopyranosyl)-(1→4)-2.6-di-O-sulfo-3-O-methyl-α-D-glucopyranosid-Natriumsalz;
- Methyl-O-(2,3,4-tri-O-methyl-6-O-sulfo-α-D-glucopyranosyl)-(1→4)-O-(2,3-di-O-methyl-β-D-glucopyranosyluronsäure)-(1→4)-O-(2,3,6-tri-O-sulfo-α-Dglucopyranosyl)-(1→4)-O-(2,7-anhydro-5-C-carboxy-6-desoxy-3-O-methyl-β-Dmannoheptopyranosyl)-(1→4)-2,3,6-tri-O-sulfo-α-D-glucopyranosid-Natriumsalz.

4. Pharmazeutische Zubereitungen enthaltend als Wirkstoff ein Pentasaccharid nach irgendeinem der Ansprüche 1 bis 3 in Form des Salzes mit einer pharmazeutisch annehmbaren Base oder in Form der Säure, in Kombination oder in Mischung mit einem inerten, nichttoxischen, pharmazeutisch annehmbaren Trägermaterial.

5. Pharmazeutische Zubereitung nach Anspruch 4 in Form von Dosiseinheiten, in denen der Wirkstoff mit mindestens einem pharmazeutischen Trägermaterial vermischt ist.

6. Zubereitung nach Anspruch 5, worin jede Dosiseinheit 0.1 bis 100 mg des Wirkstoffs enthält.

7. Zubereitung nach Anspruch 6, worin jede Dosiseinheit 0,5 bis 50 mg des Wirkstoffs enthält.

8. Verwendung des Polysaccharids nach den Ansprüchen 1 bis 3 für die Herstellung eines Arzneimittels für die Behandlung von pathologischen Zuständen. die von einer Dysfunktion der Blutgerinnung abhängen.

## Claims

1. Pentasaccharide in acidic form and its pharmaceutically acceptable salts, the anionic form of which has the formula: 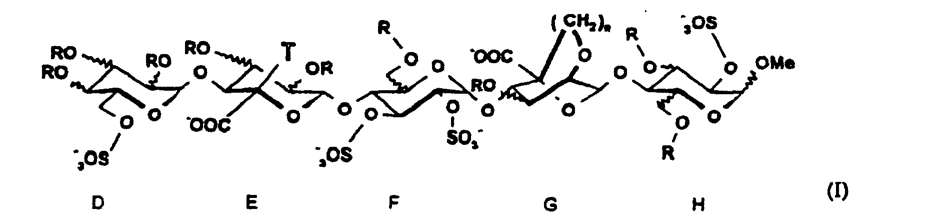 in which:
- R represents hydrogen or an -SO₃⁻, (C₁-C₃)alkyl or (C₂-C₃)acyl group;
- T represents hydrogen or an ethyl group;
- n represents 1 or 2.

2. Pentasaccharide according to Claim 1, in the form of the sodium salt or the potassium salt.

3. Pentasaccharide according to either of Claims 1 and 2, chosen from:
- Methyl O-(2,3,4-tri-O-methyl-6-0-sulpho-a-D-glucopyranosyl)-(1→4)-O-(2,3-di-O-methyl-β-D-glucopyranosyluronic acid)-(1→4)-O-(2,3,4-tri-O-sulpho-α-D-glucopyranosyl)-(1→4)-O-(2,6-anhydro-5-C-carboxy-3-0-methyl-β-D-mannopyranosyl)-(1→4)-2,3,6-tri-O-sulpho-α-D-glucopyranoside, sodium salt;
- Methyl O-(2,3,4-tri-0-methyl-6-0-sulpho-a-D-glucopyranosyl)-(1→4)-O-(5-C-ethyl-2,3-di-O-methyl-β-D-glucopyranosyluronic acid)-(1→4)-O-(2,3,6-tri-O-sulpho-α-D-glucopyranosyl)-(1→4)-O-(2,6-anhydro-5-C-carboxy-3-O-methyl-β-D-mannopyranosyl)-(1→4)-2,3,6-tri-O-sulpho-α-D-glucopyranoside, sodium salt;
- Methyl O-(2,3,4-tri-0-methyl-6-0-sulpho-a-D-glucopyranosyl)-(1→4)-O-(2,3-di-O-methyl-β-D-glucopyranosyluronic acid)-(1→4)-O-(2,3, 6-tri-O-sulpho-α-D-glucopyranosyl)-(1→4)-O-(2,6-anhydro-5-C-carboxy-3-O-methyl-β-D-mannopyranosyl)-(1→4)-2-O-sulpho-3,6-di-O-methyl-a-D-glucopyranoside, sodium salt;
- Methyl O-(2,3,4-tri-0-methyl-6-0-sulpho-a-D-glucopyranosyl)-(1→4)-O-(2,3-di-O-methyl-β-D-glucopyranosyluronic acid)-(1→4)-O-(2,3,6-tri-O-sulpho-α-D-glucopyranosyl)-(1→4)-O-(2,6-anhydro-5-C-carboxy-3-O-methyl-β-D-mannopyranosyl)-(1→4)-2,3-di-*O*-sulpho-6-O-methyl-α-D-glucopyranoside, sodium salt;
- Methyl O-(2,3,4-tri-O-methyl-6-O-sulpho-α-D-gluco-pyranosyl)-(1→4)-O-(2,3-di-O-methyl-β-D-gluco-pyranosyluronic acid)-(1→4)-O-(2,3,6-tri-O-sulpho-α-D-glucopyranosyl)-(1→4)-O-(2,6-anhydro-5-C-carboxy-3-O-methyl-β-D-mannopyranosyl)-(1→4)-2,6-di-O-sulpho-3-O-methyl-α-D-glucopyranoside, sodium salt;
- Methyl O-(2,3, 4-tri-0-methyl-6-0-sulpho-α-D-gluco-pyranosyl)-(1→4)-O-(2,3-di-O-methyl-β-D-gluco-pyranosyluronic acid)-(1→4)-O-(2,3, 6-tri-O-sulpho-α-D-glucopyranosyl)-(1→4)-O-(2,7-anhydro-5-C-carboxy-6-deoxy-3-O-methyl-β-D-mannoheptopyranosyl)-(1→4)-2,3,6-tri-O-sulpho-α-D-glucopyranoside, sodium salt.

4. Pharmaceutical compositions containing, as active principle, a pentasaccharide according to any one of Claims 1 to 3, in the form of a salt with a pharmaceutically acceptable base or in acidic form, combined with or mixed with a pharmaceutically acceptable, non-toxic inert excipient.

5. Pharmaceutical composition according to Claim 4, in the form of dosage units, in which the active principle is mixed with at least one pharmaceutical excipient.

6. Composition according to Claim 5, in which each dosage unit contains from 0.1 to 100 mg of active principle.

7. Composition according to Claim 6, in which each dosage unit contains from 0.5 to 50 mg of active principle.

8. Use of the polysaccharide according to Claims 1 to 3 for the preparation of a medicine which is useful in pathologies associated with a clotting dysfunction.
